(19) Europäisches Patentamt
European Patent Office
Office européen des brevets

(11) Publication number : 0 362 686 B1

# (12) EUROPEAN PATENT SPECIFICATION

(45) Date of publication of patent specification : **24.03.93 Bulletin 93/12**

(51) Int. Cl.$^5$ : **C07C 405/00**, // A61K31/557

(21) Application number : **89117908.7**

(22) Date of filing : **28.09.89**

## (54) Method for preparing the (+)-isomer of cloprostenol.

(30) Priority : **28.09.88 CS 6410/88**

(43) Date of publication of application : **11.04.90 Bulletin 90/15**

(45) Publication of the grant of the patent : **24.03.93 Bulletin 93/12**

(84) Designated Contracting States : **DE GB IT NL**

(56) References cited :
**FR-A- 2 137 712**
**STN FILE SEUER (KARLSRUHE) FILE CAS: CHEMICAL ABSTRACTS, vol. 111, no. 15, abstract no. 133893a, Columbus, Ohio, US; & CS-A-255 172**
**JOURNAL OF THE AMERICAN CHEMICAL SOCIETY, vol. 92:2, 28th January 1970, pages 397-398; E.J. COREY et al.: "Total synthesis of prostaglandins F2alpha and E2 as the naturally occurring forms"**
**PROSTAGLANDINS, vol. 6, no. 1, 10th April 1978, pages 87-90; D. BINDER et al.: "16-Aryloxyprostaglandins: A new class of potent luteolytic agent"**
**CA SELECTS. PROSTAGLANDIS, no. 18, September 1984, page 10, abstract no. 72513w, Columbus, Ohio, US; & CS-A-204 595 (K. CAPEK et al.) 01-06-1983**
**PROSTAGLANDINS, vol. 17, no. 6, June 1979, pages 789-800; J. BOWLER et al.: "Double bond isomers of cloprostenol"**

(73) Proprietor : **Vysoká skola chemicko-technologická**
**Suchbátarova 5**
**Praha 6 (CS)**

(72) Inventor : **Votava, Vladimir Dipl.-Ing.**
**Delnicka 65**
**Praha 7 (CS)**
Inventor : **Vesely, Ivan Dipl.-Ing. CSc.**
**Marxova 1181/6**
**Neratovice (CS)**
Inventor : **Zak, Bohumil Dipl.-Ing.,**
**Hlavni 324**
**Neratovice (CS)**
Inventor : **Dolansky, Vladimir Dipl.-Ing.**
**Jakimova 1024**
**Neratovice (CS)**
Inventor : **Drahonovsky, Jan RNDr**
**Hlavni 935**
**Neratovice (CS)**
Inventor : **Stanek, Jan Dipl.-Ing. CSc.**
**Zitna 30**
**Praha 2 (CS)**
Inventor : **Ledvinova, Marie RNDr**
**Marxova 873**
**Neratovice (CS)**
Inventor : **Capek, Antonin Dipl.-Ing.**
**Hlavni 782**
**Neratovice (CS)**
Inventor : **Mostecky, Jiri**
**Na Petynce 35**
**Praha 6 (CS)**
Inventor : **Kubelka, Vladislav RNDr CSc.**
**Amurska 1**
**Praha 10 (CS)**
Inventor : **Palecek, Jaroslav Doz.-Ing. CSc.**
**Na Hubalce 18**
**Praha 6 (CS)**
Inventor : **Svoboda Jiri Dipl.-Ing. CSc.**
**Minska 17**
**Praha 10 (CS)**

(74) Representative : **Patentanwälte Beetz sen. - Beetz jun. Timpe - Siegfried - Schmitt-Fumian-Mayr**
**Steinsdorfstrasse 10**
**W-8000 München 22 (DE)**

EP 0 362 686 B1

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The invention relates to a method for preparing the (+)- isomer of cloprostenol (1R-[1α(Z),2β(1E,3R)3α,5α-]-(+)-7-[2-(4-(3-chlorophenoxy)-3-hydroxy-1-butenyl)-3,5-dihydroxycyclopentyl]-5-heptenic acid). This substance in combination with the corresponding (-)-isomer, viz. the racemate, belongs to important analogues of prostaglandin $F_2$alpha which have found wide use in the veterinary medicine (Biologizace a chemizace zivocisne vyroby, Veterinaria-special issue, 1988; Kontakte (Merck, Darmstadt) 50, 1984; Chem. Eng. News 16, (1982) 30; CS-A-190 545 and 192 456).

A combination of the (+) and (-)-isomers bearing the generic indication of cloprostenol is commercially available and widely used in the veterinary clinical practice for the control of the ovarian functions in farm animals and particularly for producing oestrus in livestock as well as for inducing birth with sows (cf. the aforementioned references).

The preparation of some optically active substances on the basis of cloprostenol either by splitting a corresponding racemate or from optically active intermediate products has already been discussed in the literature (cf., e.g. GB-A-1 350 971; US-A-4 070 637; CS-A-190 344; JP-A-79/52 730; Prostaglandins 15 (1978) 773). According to this prior art, the preparation of pure (+)-isomer of cloprostenol in high yields and in sufficient quantities was not possible. Accordingly, the biological efficiency of the two different optically active isomers has been referred to only in a very general way. CS-A-4415-86 relates to the unexpected detection that it is only the (+)-isomer of cloprostenol that exhibits significant luteolytic effects whereas its (-)-isomer shows just antagonistic effects in this respect, and to the specific use of the (+)-isomer in the veterinary practice to control livestock oestrus and to induce birth with sows. The (+)-isomer has the further advantage of a better tolerance, particularly due to the necessity of administering only fractions of usual doses; furthermore, the (+)-isomer is also of high economical interest.

It is the object of the present invention to provide a process for the industrial production of the optically active (+)-isomer of cloprostenol of formula I

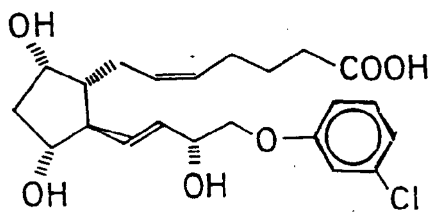

(I).

The method of the present invention starts from the prior art methods according to CS-A-204 594, 230 595, 230 236, 244 710 and 255 172.

The above object is achieved according to the claims. The dependent claims relate to preferred embodiments.

The method according to the present invention for preparing the (+)-isomer of cloprostenol of formula I is characterized by

(A) regioselective reduction of a protected optically active lactone (-)-isomer having S-configuration of the general formula II,

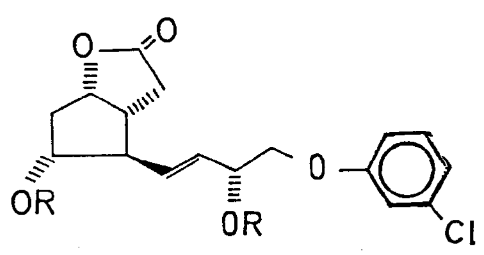

(II),

wherein R is

1-methoxybenzyl,
tetrahydropyran-2-yl,
4-methoxytetrahydropyran-2-yl or
tetrahydrofuran-2-yl,

with a reducing agent to the corresponding optically active lactol (-)isomer of formula III,

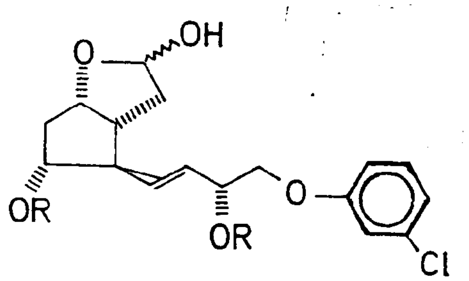

(III),

wherein R is as defined above,

(B) conversion of the lactol (-)-isomer of formula III by reacting with a phosphonium ylide prepared in situ from a 4-carboxybutyl-triphenylphosphonium halide by action of a strong base in an etheric solvent and subsequent decomposition of the reaction mixture into the corresponding protected optically active (+)-isomer of cloprostenol of formula IV,

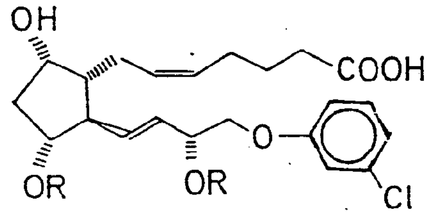

(IV),

wherein R is as defined above,

and

(C) deprotection of the protected (+)-isomer of formula IV and isolation of the (+)-isomer of cloprostenol of formula I.

According to a preferred embodiment, sodium bis(2-methoxy-ethoxy)-aluminum hydride or diisobutylaluminum hydride are used in step A as reducing agents. It is advantageous according to the present invention to use in step A a 1.5- to 2-fold molar excess of the reducing agent as compared with the amount of the compound of formula II.

In step A, the reduction is preferably carried out in a medium of a hydrocarbon having 6 to 12 carbon atoms, preferably in hexane and/or toluene, and preferably at a temperature of from -65 to -55 °C.

The ylide is preferably prepared in step B from 4-carboxybutyl-triphenylphosphonium bromide or chloride by the action of the strong base in an etheric medium, preferably diisopropylether, dibutylether, tetrahydrofurane, dioxane and/or dimethoxyethane, at a temperature of from +5 to -5 °C.

Preferred strong bases for converting the phosphonium halide into the corresponding ylide are potassium t-butylate or potassium amylate.

In accordance with another preferred embodiment, in step B, a 2.0- to 4-fold molar excess of the phosphonium ylide, based on the amount of the compound of formula III, is used.

The decomposition of the reaction mixture in step B is advantageously carried out by using an aqueous sodium hydrogen sulphate solution.

The compound of formula IV is preferably extracted, after decomposition in step B, from the reaction mixture by means of the same or another etheric solvent as that used in step B and/or an alcohol having 1 to 4 carbon atoms or mixtures thereof with water before step C.

The final deprotection of the compound of formula IV, which is preferably effected after previous removal of a portion of the solvent used for extraction of the compound of formula IV, by means of catalytic amounts of acidic agents, preferably an acidic ion exchanger in the $H^+$ cycle. In this manner, the protecting groups are removed from the protected OH-groups in positions 11 and 15 (according to the so-called prostaglandin counting). As the acidic agent there is preferably used a strongly acidic ion exchanger at room temperature or an elevated temperature. Examples for suitable strongly acidic ion exchangers are Dowex 50 and Amberlite IR-120. After the reaction, this catalyst is filtered off whereupon from the filtrate, after evaporation of the solvents, a raw product is obtained which, after purification, preferably by column chromatography, gives the optically active (+)-isomer of cloprostenol of formula I at a good yield and with high purity.

The most important advantages of the method according to the invention reside in the relatively simple reactions used, in the easy isolation of crystalline intermediate products which are easily obtainable in high,

analytic purity, and in the use of easily available, inexpensive and safe-operable agents.

The method of the present invention will hereinafter be illustrated with reference to examples.

Example 1

To a suspension of 14.2g (42 mmol) of (3aα,4β(1E,3R),5,6a)-(-)-4-[4-(3-chlorophenoxy)-3-hydroxybuten-1-yl]-5-hydroxy- 2H-hexahydrocyclopentan[b]furan-2-one (melting point 108 to 112 °C, $[\alpha]_D^{22}$= -17 ° (c = 1), $CHCl_3$) in 212 ml dichloromethane were added 3.6 ml of a 0.26 M solution of p-toluene-sulphonic acid in dichloromethane. Under external cooling of the mixture to a temperature of from -1 to +1 °C and with intensive stirring, there were added dropwise 10.1 ml (9.4 g, i.e. 112 mmol) of freshly distilled dihydropyrane. The reaction mixture was then stirred for another 20 min at the same temperature (reaction course control by thin-layer chromatography - silica gel Merck; eluent: 5 % methanol in chloroform), and after conversion of the starting substances, the reaction mixture was added with 8.3 g of sodium hydrogen carbonate, 0.8 ml of water and 4.2 g of aluminum oxide for chromatography at room temperature. After 15 min of stirring, 4.2 g of anhydrous magnesium sulphate were added to the mixture, and after obtaining a coarse precipitate (about 15 to 20 min), the separated inorganic portion was sucked off and washed twice in 20 ml of dichloromethane. From the combined organic portion the solvents were distilled off under a subatmospheric pressure in a rotary evaporator (pressure 2.6 to 5.4 kPa, bath temperature from 30 to 50 °C). After recrystallization of the distillation residue, 20.3 g of the lactone (-)-isomer of the general formula II were obtained, wherein R stands for a tetrahydropropyran-1-yl group.

To a solution of said lactone of the general formula II in 550 ml of anhydrous toluene were added after cooling down to -55 to -65 °C (nitrogen atmosphere free of oxygen traces), dropwise and under intensive stirring, 65 ml of a 1 M solution of diisobutylaluminum hydride in hexane, and, finally, the mixture was stirred at this temperature for another 30 min. The reaction was then finished by adding 42.1 ml of methanol at the same temperature. After gradual heating of the mixture to room temperature (18 to 23 °C), there were added 84.2 g of aluminum oxide for chromatography and 18 ml of water, and the mixture was stirred for another 20 to 50 min (up to the formation of a coarse sediment). The insoluble portion was then sucked off and washed twice in 120 ml of toluene; the solvents were evaporated from the combined organic portions in a rotary evaporator (pressure 2.6 to 5.4 kPa, bath temperature 30 to 50 °C). There were obtained 23.0 g (yield 97 %) of a white solid amorphous product of formula II, wherein R stands for tetrahydropyran-2-yl; melting temperature 75 to 80 °C.

Elementary analysis for $C_{27}H_{37}ClO_7$ (500.0):

| | % C | % H | % Cl |
|---|---|---|---|
| calculated: | 63.70 | 7.33 | 6.90 |
| found: | 63.65 | 7.27 | 6.98 . |

Optical rotation: $[\alpha]_D^{20}$ = 36.6° (c = 1, $CHCl_3$).

IR-spectrum (chloroform):

3300 to 3450 and 3600 $cm^{-1}$ corresponding to ν $(OH)_{assoc.}$ and to ν $(OH)_{free}$;
3020 and 3060 $cm^{-1}$ (ν $(CH)_{arom.}$);
2856, 2878, 2945 $cm^{-1}$ (ν $(CH)_{aliph.}$);
1578 and 1596 $cm^{-1}$ (ν $(C = C)_{arom.}$);
1441, 1446, 1463 and 1478 $cm^{-1}$ (δ $(CH)_{aliph.}$);
1115 $cm^{-1}$ (δ (OH));
1020, 1025, 1065, 1095 $cm^{-1}$ (ν $(C\text{-}O)_{acetal}$);

UV-spectrum in (ethanol):

$\lambda_{max}$ nm /logε[$mol^{-1} \cdot cm^{-1}$]: 220/3.13 (c = 3.5 · $10^{-4}$ M);
268/4.22; 274/4.32; 282/4.28 (c = 3.5 · $10^{-5}$ M).

A suspension of 52.0 g (117.2 mmol) of 4-carboxybutyl-triphenylphosphonium bromide in 484 ml of anhydrous tetrahydrofurane (free of peroxide traces) was added, under stirring (nitrogen atmosphere) and with external cooling to a temperature of from 15 to 25 °C, dropwise with 170.2 ml of a 1.465 M solution (149.3 mmol) of potassium t-butanolate in tetrahydrofurane within 20 to 30 min. After addition of the whole alcoholate amount

and after 20 min stirring at the same temperature the orange colored reaction mixture was cooled down to 0 °C whereupon a solution of 23 g (45.2 mmol) of the lactol of formula III was added, wherein R stands for the tetrahydropyran-2-yl group,the lactol having been dissolved in 182 ml of the above-mentioned solvent (within about 20 to 40 min) at a temperature of from 0 to 2 °C; finally, the mixture was stirred for another 2 h. Then the reaction mixture was decomposed by adding 254 ml of a saturated NaCl solution and 85 ml of cold water whereupon, after addition of 27 ml of an aqueous sodium hydrogen sulphate solution, the pH value of the mixture was adjusted to 2 - 3. After separation of the organic phase, the water layer was extracted with 85 ml of tetrahydrofurane, and the combined organic portions were washed twice in 85 ml of saturated NaCl solution. The obtained solution was then concentrated in a rotary evaporator (pressure 2.6 to 3.2 kPa, bath temperature 30 to 50 °C) to a volume of about 350 ml.

The evaporation residue was then added with 170 ml of demineralized water and 54.4 g of ion exchanger Dowex in $H^+$ cycle; the mixture was kept boiling for 2 to 6 h (the course of deprotection was monitored by thin-layer chromatography on silica gel; eluent: dioxane/methanol/chloroform 0.5 : 1 : 8.5). After the deprotection, the ion exchanger was sucked off and washed twice in 50 ml tetrahydrofurane. After cooling the filtrate to about 30 °C, its pH value was adjusted with 1 M sodium acetate solution to the exact value of 5.0. A predominant portion of solvents was evaporated in a rotary evaporator (pressure 2.6 to 3.5 kPa, bath temperature 30 to 55 °C). To the evaporation residue 24 ml of ethanol and 48 ml of toluene were added, whereupon the evaporation was repeated.

After dissolving the distillation residue in 180 ml of ethylacetate, several crystals of diphenylphosphovaleric acid were added. After a period of 24 h 3 ml of n-hexane were added; after another 24 h, 18 ml of the same solvent were added. Finally, the mixture was cooled to 0 to 5 °C and kept standing at this temperature for 2 to 4 d. The separated diphenylphosphovaleric acid was sucked off and washed in ethylacetate (3 times 36 ml). The filtrate was evaporated in a vacuum rotary evaporator to dryness. The distillation residue (49 g) containing the product was dissolved in chloroform (100 ml) and column chromatographed on silica gel (eluent: methanol/acetic acid/chloroform 2 : 0.1 : 97.9 up to 5 : 0.1 : 94.9). The product containing fractions were combined and evaporated. After a usual treatment, there were obtained 10.6 g of a light-yellow oily (+)-isomer of cloprostenol of formula I which, after having been analyzed by high-pressure liquid chromatography, contained 0.45 % of 15-epimer and 2.5 % of 5,6-trans isomer.

Elementary analysis for $C_{22}H_{29}ClO_6$ (424.9):

| | % C | % H | % Cl |
|---|---|---|---|
| calculated: | 62.19 | 6.88 | 8.34 |
| found: | 62.41 | 6.54 | 8.16 . |

Optical rotation: $[\alpha]_D^{20}$ = +22.8° (c = 1, ethanol).
IR-spectrum: See Figure 1 (measured in substance).
UV-spectrum: See Figure 2 (measured in ethanol, c = $5 \cdot 10^{-4}$).
$^1$H-NMR spectrum: See Figure 3 (measured in $CDCl_3$).
Mass spectrum: See Figure 4 (ionization by 70 eV electron impact).

## Example 2

100 mg (0.295 mmol) of (3 aα,4β(1E,3R), 5α, 6aα)-(-)-4-[4-(3-chlorophenoxy)-3-hydroxybuten-1-yl]-5-hydroxy-2H-hexahydrocyclopentan[ b]furan-2-one (melting point 108 to 112 °C, $[\alpha]_D^{22}$ = -17° (c = 1, $CHCl_3$) and 76 mg (0.75 mmol) of triethylamine were dissolved in 10 ml of 1,2-dichloroethane whereupon, after cooling the solution to a temperature of from 18 to 22 °C, 0.475 ml of a 1.4 M solution (0.665 mmol) of α-methoxybenzylchloride in 1,2-dichloroethane were added within 5 to 10 min under stirring in a nitrogen atmosphere. The reaction was monitored by thin-layer chromatography. After 15 to 20 min, the reaction was interrupted by adding 10 ml of saturated sodium hydrogen carbonate solution. The organic phase was separated, and the aqueous phase was extracted by washing twice in 3 ml of the above solvent. The organic portions were combined and dried by means of anhydrous magnesium sulphate. The solvents were evaporated under a subatomspheric pressure. From the yellow-brown viscous distillation residue were obtained, after purification with column chromatography on silica gel (eluent: chloroform/triethylamine 999 : 1), 160 mg (yield 95 %) of an oily product, and

which, according to high-pressure liquid chromatography analysis, contained 97.8 % of the product of formula II in which R stands for a $C_6H_5CH(OCH_3)$ group.
Optical rotation: $[\alpha]_D^{22}$ = -21.9° (c = 0.9, chloroform).

| | % C | % H | % Cl |
|---|---|---|---|
| calculated: | 68.45 | 6.09 | 6.12 |
| found: | 68.32 | 6.18 | 6.41 . |

IR-spectrum: (measured in substance): 3020, 365, 3095 $cm^{-1}$ ($\nu$ $(CH)_{arom.}$);
2938 $cm^{-1}$ ($\nu$ $(CH)_{aliph.}$);
2835 $cm^{-1}$ ($\nu$ (CH) in $OCH_3$ group);
1765 $cm^{-1}$ ($\nu$ (CO) in lactone);
1578 and 1592 $cm^{-1}$ ($\nu$ (C = C));
1450, 1465, 1492 $cm^{-1}$ ($\nu$ $(CH)_{aliph.}$);
1030, 170, 1105 $cm^{-1}$ ($\nu$ $(C\text{-}O)_{acetal}$).
UV-spectrum (in ethanol, c = 3.94 · $10^{-4}$ M):
$\lambda_{max}$ nm / log$\varepsilon$ [$mol^{-1}$ · $cm^{-1}$]: 251/2.87; 257/2.99; 263/3.09;
267/3.16; 274/3.26; 282/3.21.

Mass spectrum (direct evaporation into ion source; ionization by 70 eV electron impact): characteristic ion species (m/z % rel. int.)): 121(100); 105(35); 106(20); 77(38); 441 (> 1) - corresponding to M-173; 336 (> 1) corresponding to M-2x 121; 320 (> 1) corresponding to M-137-121; 304 (> 1) corresponding to M-2x 137.

A solution of 400 mg (0.69 mmol) of the bis-acetal of general formula II, wherein R stands for $C_6H_5CH(OCH_3)$, in 21 ml of toluene (nitrogen atmosphere) was added, under intensive stirring for 15 min and after cooling the solution to a temperature of from -6 to -65 °C, with 1.1 ml of a 1.2M (1.32 mmol) solution of diisobutylaluminum hydride in toluene. After 30 min stirring at the same temperature (the reaction was monitored by thin-layer chromatography), a hydride excess was decomposed by 1 ml of methanol, and after gradually heating the mixture to room temperature (about 18 °C), there were added 1.8 g of aluminum oxide for chromatography, 0.4 ml of water and after another 30 min stirring, 1.8 g of anhydrous magnesium sulphate. Within another about 20 to 30 min stirring a lumpy precipitate was obtained which was then sucked off and washed twice with 3 ml of toluene. From the combined filtrates were obtained, after evaporating the solvents, 399 mg of a product in the form of a viscous oil containing, according to high-pressure liquid chromatography analysis 98.5 % of the product of formula III (R having the above meaning).
Optical rotation: $[\alpha]_D^{22}$ = -29.1° (c = 0.5, chloroform).

Elementary analysis for $C_{33}H_{37}ClO_7$ (581.1):

| | % C | % H | % Cl |
|---|---|---|---|
| calculated: | 68.21 | 6.42 | 6.10 |
| found: | 68.44 | 6.70 | 6.13 . |

IR-spectrum: (measured in substance):
3300 to 3510 $cm^{-1}$ ($\nu$ $(OH)_{assoc.}$);
3035, 3062, 3095 $cm^{-1}$ ($\nu$ $(CH)_{arom.}$);
2940 $cm^{-1}$ ($\nu$ $(CH)_{aliph.}$);
2835 $cm^{-1}$ ($\nu$ (CH) in $CH_3O$ group);
1578 and 1592 $cm^{-1}$ ($\nu$ (C = C));
1450, 1465, 1493 $cm^{-1}$ ($\delta$ (CH)) and
1028, 1065, 1100 $cm^{-1}$ ($\nu$ $(C\text{-}O)_{acetal}$).
UV-spectrum (in ethanol, c = 4 · $10^{-4}$ M):
$\lambda_{max}$ nm / log$\varepsilon$ [$mol^{-1}$ · $cm^{-1}$]: 251/2.89; 257/3.01; 263/3.10;
267/3.16; 274/3.25; 282/3.21.

Mass spectrum (characteristic ion species (m/z (% rel.int.)): 121(100), 105(34), 106(22), 77(60), 443(> 1)

- corresponding to M-137 338 (> 1) - corresponding to M-2x 121, 322 (> 1) - corresponding to M-137-121, 306 (> 1) - corresponding to M-2x 137.

An ylide prepared from 2.6 g (5.86 mmol) of 4-carboxybutyl-triphenylphosphonium bromide in 24.2 ml of tetrahydrofurane according to Example 1 was added with 1.36 g (2.34 mmol) of the lactol of formula II (R = $C_6H_5CH(OCH_3)$) dissolved in the above-said solvent at a temperature of from -1 to +1 °C within 15 min. The mixture was then stirred for another 2 h. After an analogous treatment of the reaction mixture as referred to in Example 1, 0.679 mg of a light-yellowish (+)-isomer of cloprostenol of formula I were obtained, the physico-chemical characteristics of which were identical with those of the standard product.

## Claims

1. A method for preparing the (+)-isomer of cloprostenol of formula I,

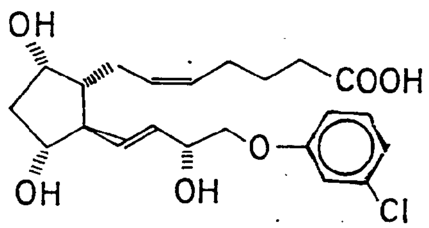

(I),

characterized by

(A) regio selective reduction of a protected optically active lactone (-)-isomer having S configuration of the general formula II,

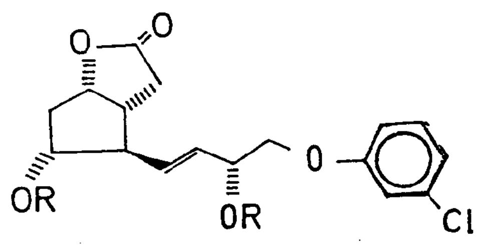

(II),

wherein R is
1-methoxybenzyl,
tetrahydropyran-2-yl,
4-methoxytetrahydropyran-2-yl or
tetrahydrofuran-2-yl,

with a reducing agent to the corresponding optically active lactol (-)isomer of formula III,

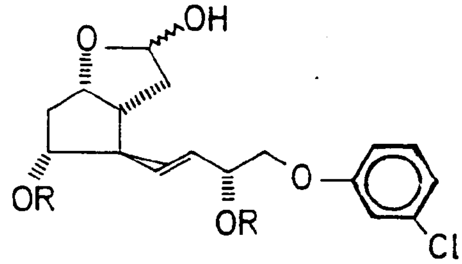

(III),

wherein R is as defined above,

(B) conversion of the lactol (-)-isomer of formula III by reacting with a phosphonium ylide prepared in situ from a 4-carboxybutyl-triphenylphosphonium halide by action of a strong base in an etheric solvent and subsequent decomposition of the reaction mixture into the corresponding protected optically active (+)-isomer of cloprostenol of formula IV,

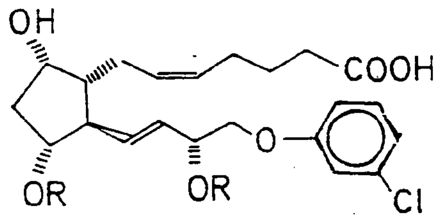

(IV),

wherein R is as defined above,
and
(C) deprotection of the protected (+)-isomer of formula IV and isolation of the (+)-isomer of cloprostenol of formula I.

2. The method according to claim 1, characterized in that in step A, sodium bis(2-methoxyethoxy)-aluminum hydride or diisobutylaluminum hydride are used as reducing agents.

3. The method according to claim 1 or 2, characterized in that in step A, a 1.5- to 2-fold molar excess of the reducing agent, based on the amount of the compound of formula II, is used.

4. The method according to one of claims 1 to 3, characterized in that in step A, the reduction is carried out in a medium of a hydrocarbon having 6 to 12 carbon atoms, preferably in hexane and/or toluene, at a temperature of from -65 to -55 °C.

5. The method according to one of claims 1 to 4, characterized in that in step B, diisopropylether, dibutylether, tetrahydrofurane, dioxane and/or 1,2-dimethoxyethane are used as etheric solvents.

6. The method according to one of claims 1 to 5, characterized in that in step B, potassium t-butylate or potassium amylate are used as strong bases.

7. The method according to one of claims 1 to 6, characterized in that in step B, a 2.0- to 4-fold molar excess of the phosphonium ylide, based on the amount of the compound of formula III, is used.

8. The method according to one of claims 1 to 7, characterized in that in step B, the reaction of the phosphonium ylide with the compound of formula III is carried out at a temperature of from -5 to +5 °C.

9. The method according to one of claims 1 to 8, characterized in that in step B, an aqueous sodium hydrogen sulphate solution is used for the decomposition of the reaction mixture.

10. The method according to one of claims 1 to 9, characterized in that after decomposition in step B, the compound of formula IV is extracted from the reaction mixture by means of the etheric solvent used in step B and/or an alcohol having 1 to 4 carbon atoms or mixtures thereof with water before step C.

11. The method according to one of claims 1 to 10, characterized in that in step C, eventually after removing a portion of the solvent used for extraction of the compound of formula IV, deprotection is effected by means of an acidic agent, preferably an acidic ion exchanger in the $H^+$-cycle.

## Patentansprüche

1. Verfahren zur Herstellung des (+)-Isomers von Cloprostenol der Formel I,

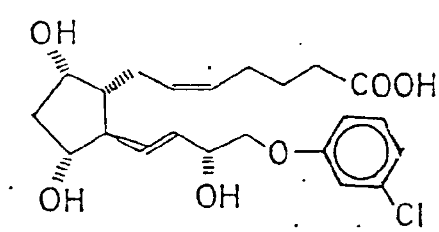

(I),

gekennzeichnet durch

(A) regioselektive Reduktion eines geschützten, optisch aktiven Lacton-(-)-Isomers mit S-Konfiguration der allgemeinen Formel II

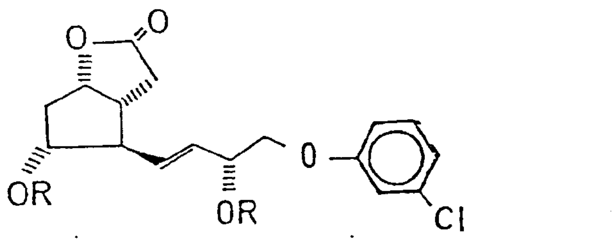

(II),

worin R

1-Methoxybenzyl,
Tetrahydropyran-2-yl,
4-Methoxytetrahydropyran-2-yl oder
Tetrahydrofuran-2-yl bedeutet,

mit einem Reduktionsmittel zum entsprechenden optisch aktiven Lactol-(-)-Isomer der Formel III,

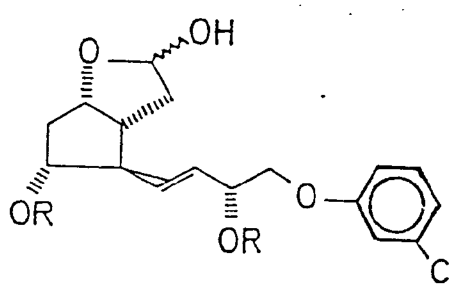

(III),

in der R die obige Bedeutung besitzt,

(B) Umwandlung des Lactol-(-)-Isomers der Formel III durch Umsetzung mit einem in situ aus einem 4-Carboxybutyltriphenylphosphoniumhalogenid durch Einwirkung einer starken Base in einem etherischen Lösungsmittel hergestellten Phosphoniumylid und anschließende Zersetzung des Reaktionsgemischs in das entsprechende geschützte, optisch aktive (+)-Isomer des Cloprostenols der Formel IV,

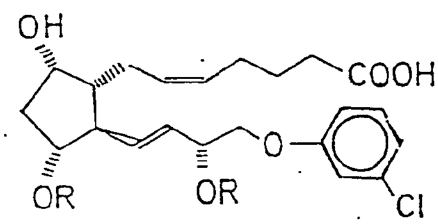

(IV),

worin R die obige Bedeutung besitzt,

und

(C) Schutzgruppenabspaltung vom geschützten (+)-Isomer der Formel IV und Isolierung des (+)-Isomers des Cloprostenols der Formel I.

**2.** Verfahren nach Anspruch 1, dadurch gekennzeichnet, daß in Schritt A als Reduktionsmittel Natrium-bis-(2-methoxyethoxy)-aluminiumhydrid oder Diisobutylaluminiumhydrid verwendet werden.

**3.** Verfahren nach Anspruch 1 oder 2, dadurch gekennzeichnet, daß in Schritt A ein 1,5- bis 2-facher molarer Überschuß des Reduktionsmittels, bezogen auf die Menge der Verbindung der Formel II, eingesetzt wird.

**4.** Verfahren nach einem der Ansprüche 1 bis 3, dadurch gekennzeichnet, daß die Reduktion in Schritt A in einem Medium eines Kohlenwasserstoffs mit 6 bis 12 C-Atomen, vorzugsweise in Hexan und/oder Toluol, bei einer Temperatur von -65 bis -55 °C durchgeführt wird.

5. Verfahren nach einem der Ansprüche 1 bis 4, dadurch gekennzeichnet, daß in Schritt B als etherische Lösungsmittel Diisopropylether, Dibutylether, Tetrahydrofuran, Dioxan und/oder 1,2-Dimethoxyethan eingesetzt werden.

6. Verfahren nach einem der Ansprüche 1 bis 5, dadurch gekennzeichnet, daß in Schritt B als starke Basen Kalium-t-butylat oder Kaliumamylat eingesetzt werden.

7. Verfahren nach einem der Ansprüche 1 bis 6, dadurch gekennzeichnet, daß in Schritt B ein 2,0- bis 4-facher molarer Überschuß des Phosphoniumylids, bezogen auf die Menge der Verbindung der Formel III, eingesetzt wird.

8. Verfahren nach einem der Ansprüche 1 bis 7, dadurch gekennzeichnet, daß die Umsetzung des Phosphoniumylids mit der Verbindung der Formel III in Schritt B bei einer Temperatur von -5 bis +5 °C durchgeführt wird.

9. Verfahren nach einem der Ansprüche 1 bis 8, dadurch gekennzeichnet, daß in Schritt B zur Zersetzung des Reaktionsgemischs eine wäßrige Natriumhydrogensulfatlösung verwendet wird.

10. Verfahren nach einem der Ansprüche 1 bis 9, dadurch gekennzeichnet, daß die Verbindung der Formel IV nach der Zersetzung in Schritt B mit dem in Schritt B verwendeten etherischen Lösungsmittel und/oder einem Alkohol mit 1 bis 4 C-Atomen oder Gemischen davon mit Wasser vor Schritt C aus dem Reaktionsgemisch extrahiert wird.

11. Verfahren nach einem der Ansprüche 1 bis 10, dadurch gekennzeichnet, daß die Schutzgruppenabspaltung in Schritt C, ggfs. nach Abtrennung eines Teils des zur Extraktion der Verbindung der Formel IV verwendeten Lösungsmittels, mit einem sauren Mittel, vorzugsweise einem sauren Ionenaustauscher in der $H^+$-Form, vorgenommen wird.

## Revendications

1. Procédé de préparation de l'isomère(+) du cloprosténol de formule I,

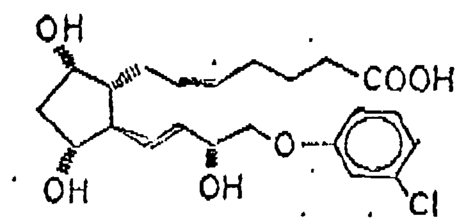

(I),

caractérisé par

(A) la réduction régiosélective de l'isomère(-) protégé, optiquement actif de lactone ayant une configuration S de formule générale II,

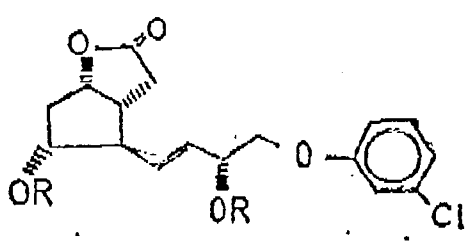

(II),

dans laquelle R représente

1-méthoxybenzyl,
tétrahydropyran-2-yl,
4-méthoxytetrahydropyran-2-yl ou
tétrahydrofuran-2-yl,

à l'aide d'un agent réducteur pour fournir l'isomère(-) optiquement actif de lactol correspondant de formule III,

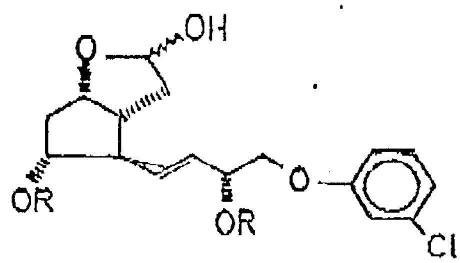

(III),

dans laquelle R est défini comme ci-dessus,
(B) la conversion de l'isomère(-) du lactol de formule III par réaction avec un ylide de phosphonium préparé in-situ à partir d'un halogénure de 4-carboxybutyl-triphénylphosphonium par action d'une base forte dans un solvant du type éther et décomposition subséquente du mélange réactionnel en l'isomère(+) protégé, optiquement actif, correspondant du cloprosténol de formule IV,

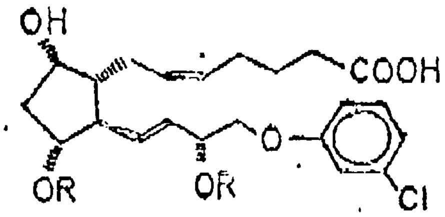

(IV),

dans laquelle R est défini comme ci-dessus, et
(C) la suppression de la protection de l'isomère(+) protégé de formule IV et l'isolement de l'isomère(+) du cloprosténol de formule I.

2. Procédé selon la revendication 1, caractérisé en ce que dans l'étape A l'hydrure de sodium bis(2-méthoxyéthoxy)aluminium ou l'hydrure de diisobutylaluminium sont utilisés comme agents réducteurs.

3. Procédé selon la revendication 1 ou 2, caractérisé en ce que dans l'étape A on utilise un excès 1,5 à 2 fois molaire d'agents réducteurs par rapport à la quantité de composé de formule II.

4. Procédé selon l'une des revendications 1 à 3, caractérisé en ce que l'étape A la réduction est effectuée au sein d'un milieu constitué par un carbure d'hydrogène ayant de 6 à 12 atomes de carbone, de préférence dans l'hexane et/ou le toluène, à une température de -65 à -55°C,

5. Procédé selon l'une des revendications 1 à 4, caractérisé en ce que dans l'étape B on utilise comme solvant du type éther le diisopropyléther, le dibutyléther, le tétrahydrofurane, le dioxane et/ou le 1,2-diméthoxyéthane.

6. Procédé selon l'une des revendications 1 à 5, caractérisé en ce que dans l'étape B on utilise en tant que base forte du t-butylate de potassium ou de l'amylate de potassium.

7. Procédé selon l'une des revendications 1 à 6, caractérisé en ce que dans l'étape B on utilise un excès 2,0 à 4 fois molaire de l'ylide de phosphonium par rapport à la quantité de composé de formule III.

8. Procédé selon l'une des revendications 1 à 7, caractérisé en ce que dans l'étape B la réaction de l'ylide de phosphonium avec le composé de formule III est effectuée à une température de -5 à +5 °C.

9. Procédé selon l'une des revendications 1 à 8, caractérisé en ce que dans l'étape B on utilise une solution acqueuse d'hydrogénosulfate de sodium pour la décomposition du mélange réactionnel.

10. Procédé selon l'une des revendications 1 à 9, caractérisé en ce qu'après la décomposition dans l'étape B le composé de formule IV est extrait du mélange réactionnel au moyen du solvant de type éther utilisé

dans l'étape B et/ou d'un alcool ayant de 1 à 4 atomes de carbone ou des mélanges de ce dernier avec de l'eau avant l'étape C.

**11.** Procédé selon l'une des revendications 1 à 10, caractérisé en ce que dans l'étape C, éventuellement après enlèvement d'une partie du solvant utilisé pour l'extraction du composé de formule IV, la suppression de la protection est effectuée au moyen d'un agent acide, de préférence d'un échangeur d'ions acide sous forme hydrogène $H^+$.

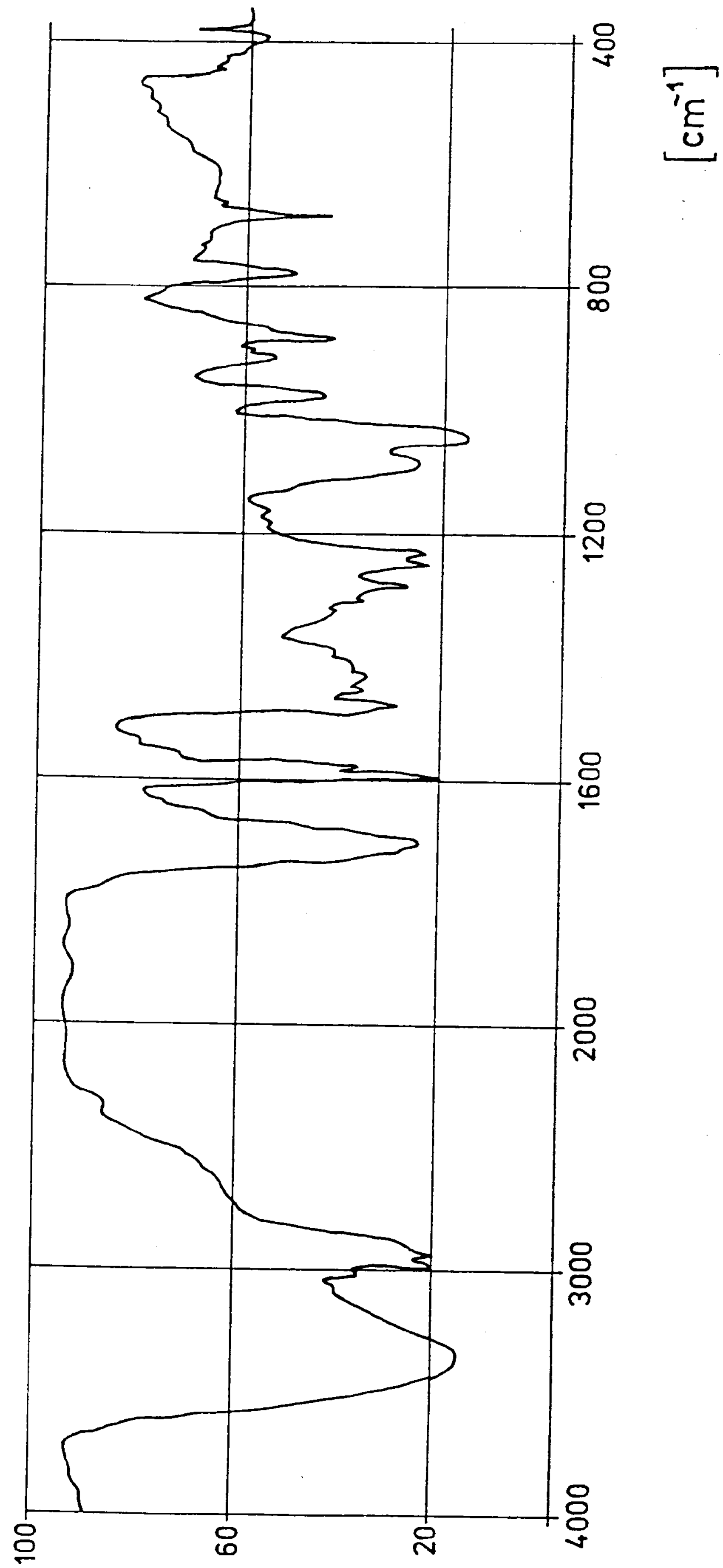


FIG. 1

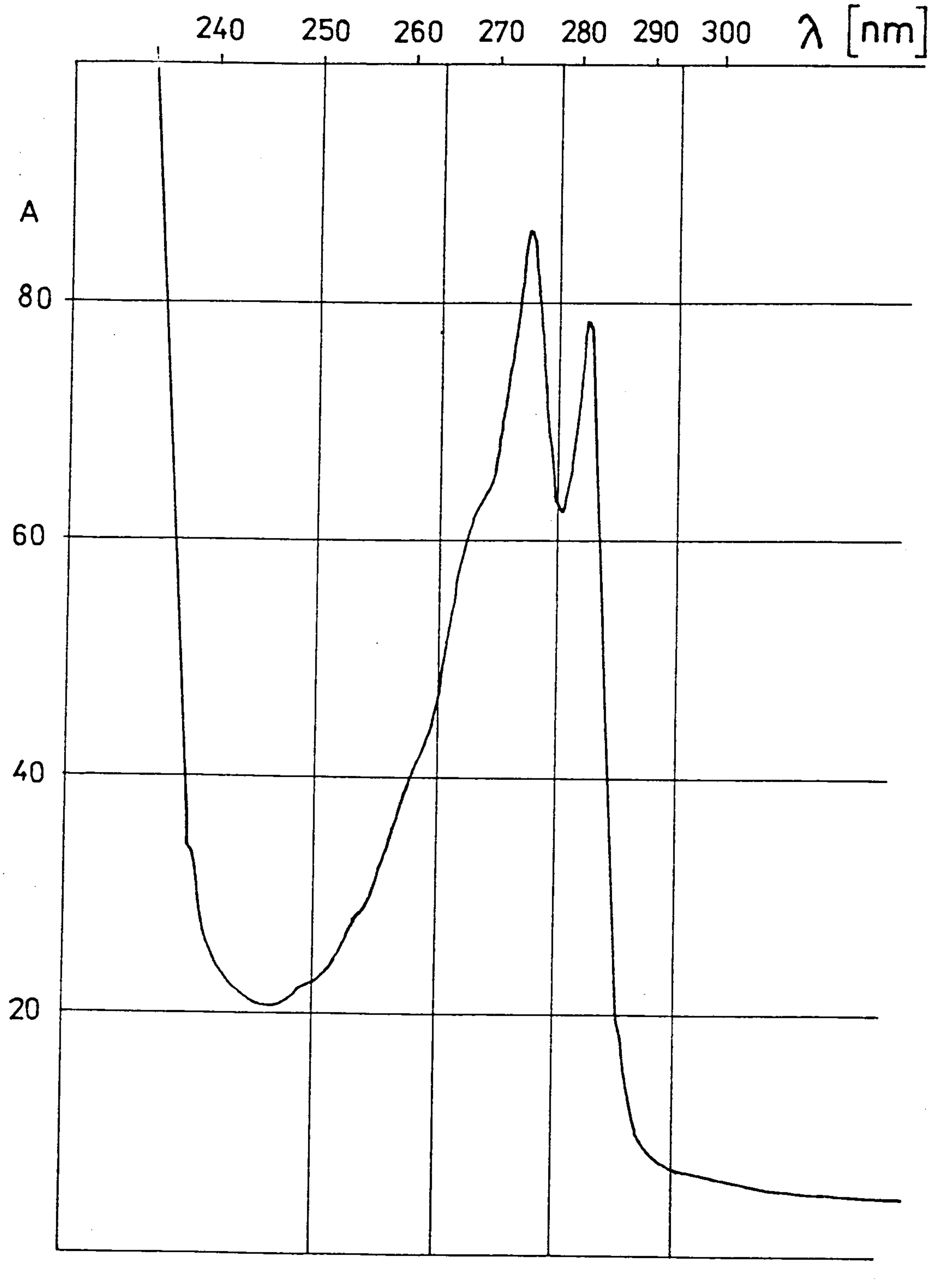


FIG. 2

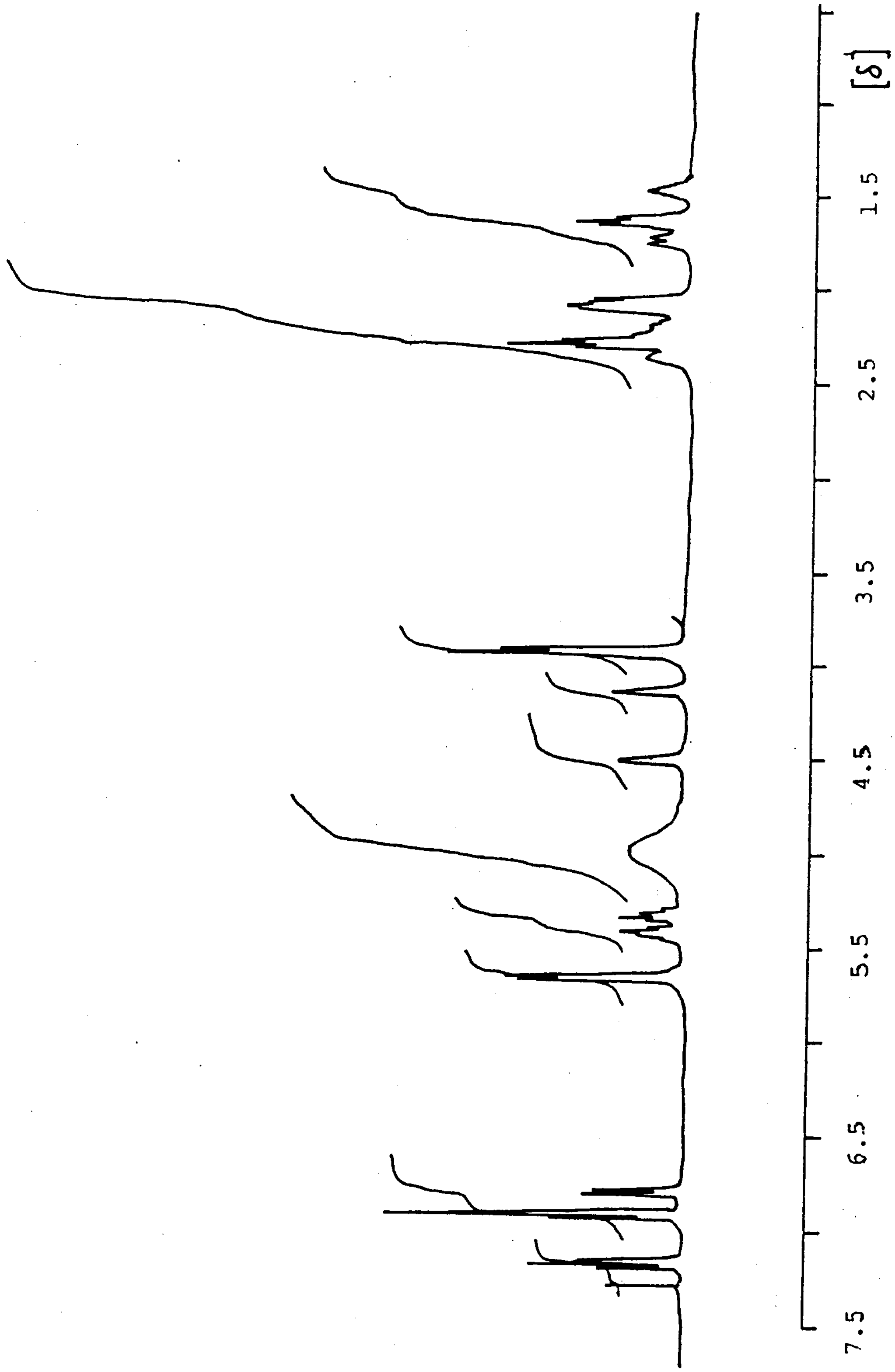


FIG.3

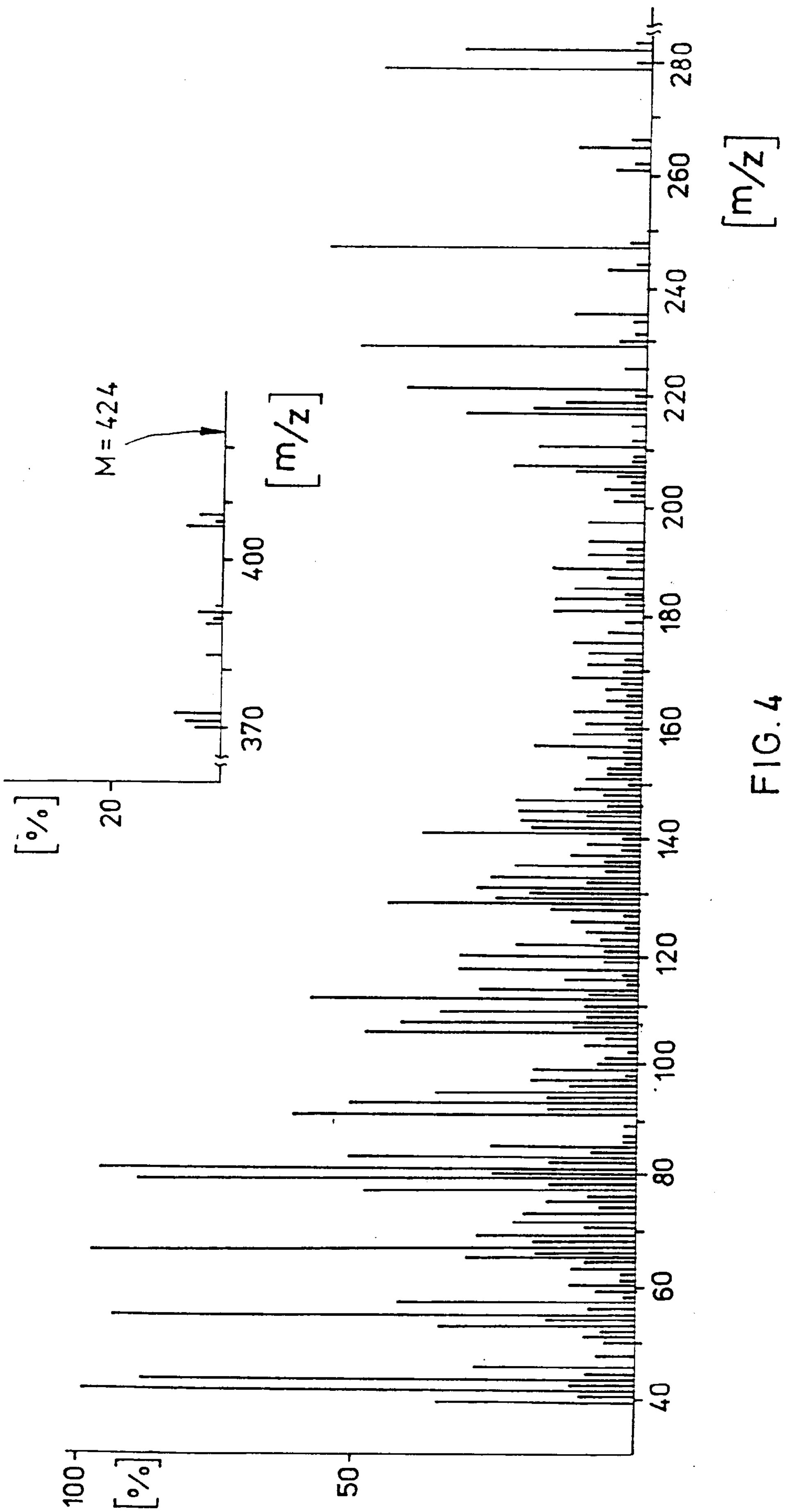

100
50
[%]
20
[%]
40
60
80
100
120
140
160
180
200
220
240
260
280
[m/z]
370
400
[m/z]
M = 424


FIG. 4